# EUROPÄISCHE PATENTANMELDUNG

(11) **EP 1 798 546 A1**
(43) Veröffentlichungstag der Anmeldung: **20.06.2007**
(21) Anmeldenummer: 06024496.9
(22) Anmeldetag: 27.11.2006
(51) Int. Cl.: G01N 27/411, G01N 33/20

(54) **Messsonde zur Messung in Metall- oder Schlackeschmelzen**

(30) Priorität: 15.12.2005 DE 102005060492
(71) Anmelder: Heraeus Electro-Nite International N.V., 3530 Houthalen (BE)
(72) Erfinder: Neyens, Guido Jacobus, 3680 Opoeteren (BE)
(74) Vertreter: Kühn, Hans-Christian

(57) **Zusammenfassung**

Die Erfindung betrifft eine Messsonde zur Messung in Metall- oder Schlackeschmelzen mit einem Messkopf, welcher ein Eintauchende und ein rückseitiges Ende aufweist, wobei am Eintauchende Sensoren angeordnet sind mit Signalleitungen, die durch Kanäle durch den Messkopf geführt sind, wobei für jeden Sensor ein separater Kanal vorgesehen ist.

## Beschreibung

Die Erfindung betrifft eine Messsonde zur Messung in Metall- oder Schlackeschmelzen mit einem Messkopf, welcher ein Eintauchende und ein rückseitiges Ende aufweist, wobei am Eintauchende Sensoren angeordnet sind.

Derartige Messsonden sind in der Metallurgie üblich und beispielsweise aus DE 35 41 806 C1 bekannt. In dieser Druckschrift ist offenbart, mehrere Sensoren und eine Probenkammer in bzw. an einen Messkopf anzuordnen. In DE 8317643 U1 ist eine ähnliche Messsonde offenbart. Auch hier sind unterschiedliche Sensoren und optional eine Probenkammer in bzw. an einem Messkopf angeordnet. Die Sensoren sind an der Eintauchseite des Messkopfes in diesen hineingesteckt, die Signalleitungen werden in separate Bohrungen eingefädelt ist durch diese hindurch nach hinten, durch ein Trägerrohr hindurch zu einer Auswerteeinheit geführt. Eine weitere Messsonde ist aus DE 7925016 U1 bekannt. Auch hier sind mehrere Sensoren an einem Messkopf angeordnet und in diesen mittels eines feuerfesten Zements fixiert.

Der Erfindung liegt die Aufgabe zugrunde, die bekannten Messsonden zu verbessern, insbesondere hinsichtlich einer einfachen und kostengünstigen Montage.

Die Aufgabe wird durcheine Messsonde gemäß den unabhängigen Ansprüchen gelöst. Vorzugsweise Ausgestaltung sind in den Unteransprüchen angegeben.

Eine Messsonde mit einem Messkopf, welcher ein Eintauchende und ein rückseitiges Ende aufweist, wobei am Eintauchende Sensoren Sensoren angeordnet sind, weist erfindungsgernäß vor seinem rückseitigen Ende seitliche Nischen auf. Insbesondere kann der Messkopf einen seitlich umlaufenden Kragen aufweisen und ein dem Eintauchende abgewandtes Ende, dessen Durchmesser kleiner ist als der Durchmesser des Kragens. Auf dieses Ende kann ein Träger rohr aufgesteckt werden. In den Nischen können Anschlüsse für Sensoren auf einfache Weise angeordnet sein, wobei die Nischen auf der dem Eintauchende angewandten Seite des Kragens in dem Messkopf angeordnet sind. Die Nischen können vorzugsweise jeweils durch mindestens einen Kanal mit dem eintauchseitigen Ende des Messkopfes verbunden sein. Durch die Kanäle hindurch kann die Montage von Sensoren an der Eintauchseite des Messkopfes erfolgen, wobei die Signalleitungen durch die Nischen hindurch angeführt werden. Die Nischen und die nach hinten abgeleiteten Signalkanal sind innerhalb eines Trägerrohres angeordnet. Dadurch ist eine sehr einfache Montage der Sensoren von nur einer Seite des Messkopfes aus möglich.

In einer weiteren Ausführungsform der erfindungsgemäßen Messsonde weist der Messkopf ein Eintauchende und ein rückseitiges Ende auf wobei am Eintauchende Sensoren angeordnet sind mit Signalleitungen, die durch Kanäle durch den Messkopf geführt sind, wobei für jeden Sensor ein separater Kanal vorgesehen ist. Die Messsonde ist dadurch gekennzeichnet, dass an dem dem Eintauchende abgewandten Ende eines Kanals die jeweiligen Signalleitungen des dem Kanal zugehörigen Sensors mit einem Kontaktstück verbunden sind. Das Kontaktstück wiederum wird mit weiteren Signalleitungen verbunden, die durch ein Trägerrohr der Messsonde hindurch zu einer Auswerteeinrichtung geführt sind. Die Montage erfolgt jeweils von der Rückseite des Messkopfes her, so dass ein Umdrehen des Messkopfes während der Montage nicht nötig ist. In jedem Kanal wird ein spezieller Sensor angeordnet, die einzelnen Sensoren sind auf diese Weise mit einem ausreichenden Abstand voneinander angeordnet, beeinflussen sich während der Messung praktisch nicht gegenseitig und können im einzelnen auf die speziellen Bedürfnisse angepasst werden, so dass ein universellen Messkopf je nach Notwendigkeit und Einsatzbestimmung mit unterschiedlichen Sensoren ausgerüstet werden kann. Während der Montage können aus Gründen der Vereinfachung dabei zunächst alle Messköpfe mit dem für alle gleichermaßen einzusetzenden Sensoren bestückt werden und anschließend gruppenweise mit den jeweils zutreffenden speziellen Sensoren für einen bestimmten Anwendungszweck. Durch eine solche Modulbauweise können die Montagekosten erheblich gesenkt werden. Mittels des Kontaktstückes kann zum einen der Kanal verschlossen und zum anderen der jeweilige Sensor in seinem Kanal gehalten werden, bevor nach Bestückung der Kanäle diese gemeinsam mit Zement oder einem anderen feuerfesten Material von der Eintauchseite her gefüllt werden, so dass die Sensoren dann fixiert sind. In einem derartigen Kanal kann auch ein Probennehmer oder ein Einlaufkanal eines am rückseitigen Ende des Messkopfes angeordneten Probennehmers angeordnet sein.

Durch die Anordnung der sog. Kontaktstücke am rückseitigen Ende des Messkopfes wird bei der Montage des Messkopfes das Hantieren mit langen Signalleitungen vermieden, wobei es insbesondere zweckmäßig ist, in jeweils einem Kanal jeweils ein Kontaktstück anzuordnen und diese an dem dem Eintauchende angewandten Ende des Kanals zu fixieren. Die spätere Montage der weiterführenden Signalleitungen wird dadurch einfacher und fehlerfrei, da eine eindeutige Zuordnung der jeweiligen Signalkabel zu dem zugehörigen Kontaktstück beispielsweise durch entsprechende farbige Markierung Fehler nahezu ausschließt. Zweckmäßig ist es, dass das dem Eintauchende abgewandte Ende eines Kanals in eine seitlich an dem Messkopf angeordnete Nische mündet. Auf diese Weise werden aus der Außenkontur des Messkopfes hervorstehende Strukturen im wesentlichen vermieden, so dass Beschädigungen während der Montage vermieden werden. Die Nischen können bei der Montage eines Trägerrohrs am Messkopf von diesem Trägerrohr abgedeckt werden.

Zweckmäßigerweise ist der Kanal als Bohrung ausgebildet und weist einen größeren Durchmesser auf als der Durchmesser des zugehörigen Sensors. Der Durchmesser der Bohrungen kann auch standarisiert sein, so dass die Flexibilität des Einsatzes von Sensoren steigt. Der Sensor kann von dem dem Eintauchende abgewandten Ende des Kanals in diesen eingeführt werden bis das Kontaktstück; das über ein relativ kurzes Signalkabel mit dem Sensor verbunden ist, an dem Kanalende anschlägt. Das Kontaktstück kann vorteilhafter Weise in ein Kanalende gesteckt sein. Dabei ist es insb. vorteilhaft, dass die Kontaktstücke an ihrem dem Eintauchende angewandten Ende einen Durchmesser aufweisen, der größer ist als der Durchmesser des jeweils zugeordneten Kanalendes, da dadurch ein Anschlag gebildet wird, bis zu dem das Kontaktstück in den Kanal hineingesteckt werden kann.

Der Messkopf kann einen Kragen aufweisen, dessen dem Eintauchende angewandtes Ende einen Anschlag für das Trägerrohr aufweist. Üblicherweise sind derartige Messköpfe im wesentlichen zylindrisch ausgebildet bzw. bestehen aus einer in Achsrichtung aneinander gereihten Vielzahl von Zylindern mit nach hinten (zur dem Eintauchende angewandten Seite hin) geringer werdenden Durchmesser. In einem solchen Fall entstehen umlaufende Abstufungen. Es ist zweckmäßig, dass unter Benutzung einer solchen Abstufung innerhalb des Trägerrohres ein weiteres Stützrohr an dem Messkopf angeordnet ist. Dies kann vorteilhaft sein bei einer Probennahme, da in der Metallschmelze das Trägerrohr im wesentlichen verbrennt und seine Stabilität verliert, so dass das darin angeordnete Stützrohr eine zusätzliche Trägerfunktion für die Probenkammer ausübt. Dieses innere Stützrohr wird nach Herausnahme der Messsonde aus der Metallschmelze und nach Entfernung der Reste des Trägerrohres von dem Messkopf entfernt, so dass die Probenkammer gut zugänglich und auf einfache Weise entnehmbar ist.

Vorteilhafter Weise ist an dem Messkopf also eine Probenkammer und/oder zweckmäßiger Weise auch ein Badkontakt angeordnet, Der Badkontakt ragt aus dem eintauchseitigem Ende des Messkopfes heraus.

Sinnvoller Weise können die Kontakte des Kontaktstückes als Steckkontakte ausgebildet sein.

Nachfolgend wird ein Ausführurigsbeispiel der Erfindung an Hand einer Zeichnung beschrieben. In der Zeichnung zeigt::
- Fig. 1: den Schnitt durch einen Messkopf und
- Fig. 2: die schematische Darstellung einer Messsonde mit Trägerrohr.

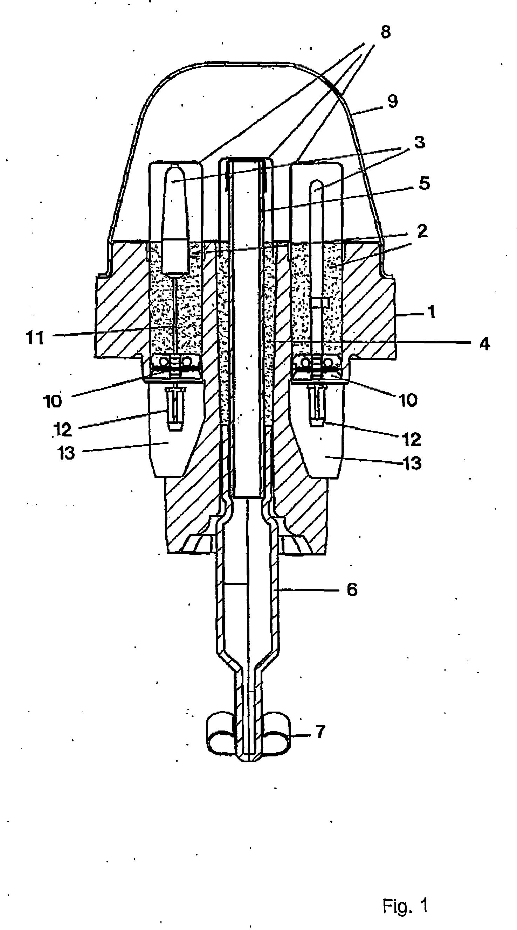

In Fig. 1 ist ein Messkopf 1 einer Messsonde dargestellt. Die Messsonde wird beispielsweise in Stahlwerken eingesetzt zum Messen von Kennzahlen der flüssigen Stahl-oder Schlackeschmelze. Der Messkopf weist mehrere Kanäle 2 auf. In diese Kanäle sind um eine Längsachse des Messkopfes 1 herum angeordnet. In ihnen sind Sensoren 3 (elektrochemische Sensoren, Temperatursensoren oder andere gebräuchliche Sensoren) angeordnet. Ein zentrisch angeordneter Kanal 4 trägt ein Einlaufrohr 5 für eine Probenkammer 6. Die Probenkammer 6 ist an dem Eintauchende angewandten Ende des Messkopfes 1 fixiert. In Fig. 1 ist ein typischer habschaliger Probennehmer dargestellt, dessen rückseitiges Ende mit einer Klammer 7 zusammengehalten wird. Die Kanäle 2; 4 sind mit feuerfestem Zement gefüllt, so dass das Einlaufrohr 5 bzw. die Sensoren 3 fixiert sind. Das über das Eintauchende des Meßkopfes 1 hervorstehend Ende der Sensoren 3 bzw. des Einlaufrohres 5 ist jeweils miteiner separaten und auf den konkreten Zweck angepassten Schutzkappe 8 abgedeckt. Die Schutzkappen 8 schützen die Sensoren 3 bzw. das Einlaufrohr 5 beim Durchtauchen durch die Schlackeschmeize. Der gesamte Messkopf 1 ist an seinem Eintauchende mit einer einzigen zusätzlichen Kappe 9 abgedeckt, die stabiler ist und die darunter liegenden Schutzkappen 8 beim Transport oder beim Durchstoßen der verkrusteten Schlacke schützt.

Die Sensoren 3 sind entweder direkt oder über Signalleitungen 11 mit jeweils einem Kontaktstück 10 verbunden. In Fig. 1 ist links ein Sauerstoffsensor und rechts ein Thermoelement dargestellt. Die Kontaktstücke 10 verschließen das rückseitige Ende der Kanäle 2. Die Kontaktstücke 10 weisen auf ihren rückseitigen Enden Steckkontakte 12 auf, die in Nischen 13 des rückseitigen Endes des Messkopfes 1 angeordnet sind. Die Nischen 13 wurden bei der Herstellung des Messkopfes 1 (wie auch wie die Kanäle) als Teil des Formteiles auf bekannte Weise gefertigt, sie können jedoch auch nachträglich gefräst werden.

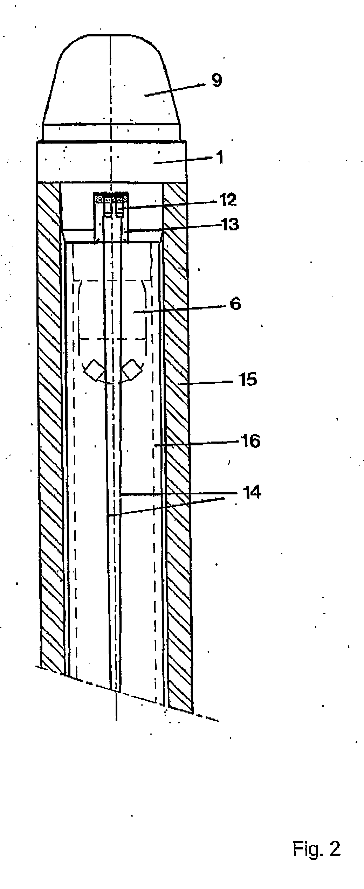

Fig. 2 zeigt den mit einer äußeren Kappe 9 abgedecken Messkopf 1, an dessen rückseitigem Ende innerhalb einer Nische 13 Steckkontakte 12 angeordnet sind. Die Steckkontakte 12 sind mit Signalleitungen 14 verbunden, die wiederum durch ein Trägerohr 15 hindurchgeführt und mit einer Auswerteelektronik verbunden sind. Auf einem zweiten, verringertem Durchmesser des rückwärtigem Ende des Messkopfes 1 ist ein Stützrohr 16 angeordnet, das die Probenkammer 6 zusätzlich schützt und eine einfache Entnahme der Probenkammer 6 nach der Probennahme sicherstellt.

Die einzeln montierten Sensoren 3 beeinflussen sich einander praktisch nicht und können auf den speziellen Verwendungszweck angestellt werden. Der Probennehmer ist effektiv vor dem Eindringen von Schlacke geschützt, da die Dimensionierung der Schutzkappen so eingestellt werden kann, dass der Einlauf des Einlaufrohres 5 erst in der Stahlschmelze freigegeben wird. Die Montage sämtlicher Elemente des Messkopfes 1 (Sensoren 3, Probenkammer 6) erfolgt von einer Seite des Messkopfes 1, das Vergießen mit feuerfestem Zement von der anderen Seite, so dass der Messkopf 1 während der Montage leicht handhabbar ist und nicht ständig gedreht werden muss. Alle Verbindungen sind gut sichtbar und können daher während der Produktion überprüft werden, längere Drähte, die während der Montage des Messkopfes 1 stören könnten, sind nicht vorhanden und die Zahl der notwendigen Brennvorgänge zum Brennen des feuerfesten Zements wird minimiert.

## Patentansprüche

1. Messsonde zur Messung in Metall- oder Schlackeschmelzen mit einem Messkopf, welcher ein Eintauchende und ein rückseitiges Ende aufweist, wobei am Eintauchende Sensoren angeordnet sind mit Signalleitungen, die durch Kanäle durch den Messkopf geführt sind, wobei für jeden Sensor ein separater Kanal vorgesehen ist, **dadurch gekennzeichnet, dass** an dem dem Eintauchende abgewandten Ende eines Kanals die Signalleitungen des zugehörigen Sensors mit einem Kontaktstück verbunden sind und dass jedes Kontaktstück an jeweils einem Kanal angeordnet und an dem dem Eintauchende abgewandten Ende des Kanals fixiert ist.

2. Messsonde nach Anspruch 1, **dadurch gekennzeichnet, dass** das dem Ein-tauchende abgewandte Ende eines Kanals in eine seitlich an dem Messkopf angeordnete Nische mündet.

3. Messsonde nach Anspruch 1 oder 2, **dadurch gekennzeichnet, dass** der Kanal als Bohrung ausgebildet ist mit einem größeren Durchmesser als der Durchmesser des zugehörigen Sensors.

4. Messsonde nach einem der Ansprüche 1 bis 3, **dadurch gekennzeichnet, dass** die Kontaktstücke jeweils in ein Kanalende gesteckt sind.

5. Messsonde nach Anspruch 4, **dadurch gekennzeichnet, dass** die Kontaktstücke an ihrem dem Eintauchende abgewandten Ende einen Durchmesser aufweisen, der größer ist als der Durchmesser des jeweils zugeordneten Kanalendes.

6. Messsonde nach einem der Ansprüche 1 bis 5, **dadurch gekennzeichnet, dass** der Messkopf in einer Öffnung eines Trägerrohres angeordnet ist.

7. Messsonde nach Anspruch 6, **dadurch gekennzeichnet, dass** innerhalb des Trägerrohres ein weiteres Stützrohr an dem Messkopf angeordnet ist.

8. Messsonde nach einem der Ansprüche 1 bis 7, **dadurch gekennzeichnet, dass** am Messkopf zusätzlich ein Badkontakt und/oder eine Probenkammer angeordnet sind.

9. Messsonde nach einem der Ansprüche 1 bis 8, **dadurch gekennzeichnet, dass** die Kontakte der Kontaktstücke als Steckkontakte ausgebildet sind.
